# EUROPÄISCHE PATENTANMELDUNG

(11) **EP 1 067 124 A1**
(43) Veröffentlichungstag der Anmeldung: **10.01.2001**
(21) Anmeldenummer: 00810570.2
(22) Anmeldetag: 30.06.2000
(51) Int. Cl.: C07D 279/20, C07D 279/36, C07C 209/60

(54) **Verfahren zur Herstellung eines Gemisches von alkylierten Phenothiazinen und Diphenylaminen**

(30) Priorität: 09.07.1999 CH 128099
(71) Anmelder: Ciba Specialty Chemicals Holding Inc., 4057 Basel (CH)
(72) Erfinder: Evans, Samuel, 1723 Marly (CH); Allenbach, Stephan, 4334 Sisseln (CH); Dubs, Paul, 6330 Cham (CH)

(57) **Zusammenfassung**

Die Erfindung betrifft ein neues, technisch vorteilhaftes Verfahren zur Herstellung eines Gemisches von alkylierten Phenothiazinen und Diphenylaminen. Ausgehend von Diphenylamin setzt man dieses mit elementarem Schwefel in Gegenwart von Jod um und behandelt das Stoffgemisch aus Diphenylamin und Phenothiazin in Gegenwart eines sauren Katalysators mit einem Olefin.

## Beschreibung

Die Erfindung betrifft ein neues, technisch vorteilhaftes Verfahren zur Herstellung eines Gemisches von alkylierten Phenothiazinen und Diphenylaminen.

Zahlreichen organischen Produkten, die in der Technik eine breite Anwendung finden, z. B. Schmierstoffen, Hydraulikflüssigkeiten, Metallbearbeitungsflüssigkeiten, Treibstoffen oder Polymeren, werden Additive zur Verbesserung ihrer Gebrauchseigenschaften zugesetzt.

Flüssige Mischungen aminischer Antioxidantien werden seit längerem insbesondere in Schmierölen für Verbrennungsmotoren eingesetzt. Aus der *U.S. Patentschrift 2,433,658* ist bekannt, durch Umsetzung von Diphenylamin mit Schwefel Phenothiazin herzustellen. In der *EP-A-0 475 904* ist die Herstellung eines Stoffgemisches aus alkylierten Phenothiazinen durch Umsetzung von alkylierten Diphenylaminen mit Schwefel beschrieben. Das alkylierte Diphenylamingemisch wird zuvor gemäss dem in der *EP-A-0 149 422* beschriebenen Verfahren durch Umsetzung von Diphenylamin mit einem Olefin als Alkylierungsmittel, z. B. Diisobutylen, hergestellt. Dieses Alkylierungsverfahren ist wenig selektiv, da Mischungen mit unterschiedlich alkylierten Produkten, z. B. Gemische von 2,2'-, 2,4'-, 4-, 4,4'- und 2,4,4'-alkylierten Diphenylaminen, erhalten werden. Die anschliessende Umsetzung solcher Mischungen nach dem in *der EP-A-0 475 904* beschriebenen Verfahren ergibt Mischungen von entsprechend alkylierten Diphenylaminen und Phenothiazinen mit geringer Selektivität.

Der vorliegenden Erfindung liegt die allgemeine Aufgabenstellung zugrunde, Mischungen von alkylierten Diphenylaminen und Phenothiazinen mit erhöhter Selektivität der Alkylierung in 3- und 7-Stellung herzustellen. In der *EP-A-0 659 749* ist die selektive Alkylierung einer Mischung von Diphenylaminen mit Phenothiazinen mit Olefinen, z. B. Diisobutylen, beschrieben. Dieses Verfahren ist jedoch nachteilig, da die Synthese als Ausgangsmaterial bereits vorhandene Gemische von Diphenylamin mit Phenothiazin voraussetzt.

Der vorliegenden Erfindung liegt die engere Aufgabenstellung zugrunde, ausgehend von reinem Diphenylamin bzw. Naphthylamin als alleinigem Ausgangsmaterial Mischungen von alkylierten Diphenylaminen und Phenothiazinen mit erhöhter Selektivität der Alkylierung in 3-und 7-Stellung herzustellen.

Die Aufgabenstellung wird durch die vorliegende Erfindung gelöst, welche ein sogenanntes Eintopfverfahren zur Herstellung eines Gemisches von alkylierten Phenothiazinen und Diphenylaminen betrifft.

Gegenstand der Erfindung ist ein Verfahren zur Herstellung eines Gemisches enthaltend alkylierte Phenothiazine der Formel: 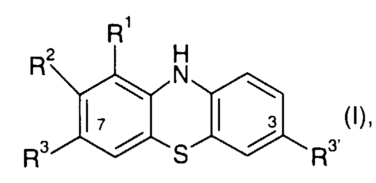 und entsprechend substituierte Diphenylamine der Formel: 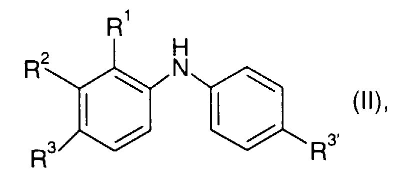 worin R¹ und R² Wasserstoff oder zusammen die Gruppe: 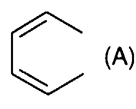 darstellen;
einer der Reste R³ und R^{3'} Wasserstoff und der andere C₂-C₃₀-Alkyl, Cyclo-C₅-C₁₂-alkyl-C₂-C₄-alkyl, α-C₁-C₂-Alkylbenzyl oder α,α-Dimethylbenzyl bedeutet;
oder beide Reste R³ und R^{3'} C₂-C₃₀-Alkyl, Cyclo-C₅-C₁₂-alkyl-C₂-C₄-alkyl, α-C₁-C₂-Alkylbenzyl oder α,α-Dimethylbenzyl bedeuten, wenn R¹ und R² Wasserstoff darstellen; oder R³ Wasserstoff und R³ C₂-C₃₀-Alkyl, Cyclo-C₅-C₁₂-alkyl-C₂-C₄-alkyl, α-C₁-C₂-Alkylbenzyl oder α,α- Dimethylbenzyl bedeuten, wenn R¹ und R² zusammen die Gruppe A darstellen, dadurch gekennzeichnet, dass man ein Diphenylamin der Formel: 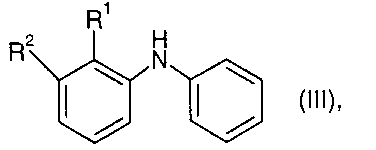 worin R¹ und R² die unter der Formel I genannten Bedeutungen haben, mit elementarem Schwefel in Gegenwart eines Kondensationskatalysators aus der Gruppe Jod, Aluminiumbromid, Aluminiumchlorid, Eisen-III-Chlorid, Antimonchlorid, Kupferiodid und Schwefeliodid umsetzt und das erhältliche Stoffgemisch von Diphenylamin (III) und Phenothiazin der Formel: 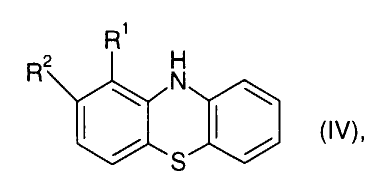 worin R¹ und R² die unter der Formel I genannten Bedeutungen haben, mit einem die Anzahl der C-Atome in R³ oder R^{3'} enthaltenden Olefin der Formel: 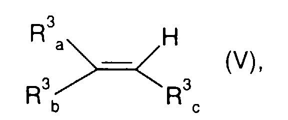 worin einer der Reste R³ₐ, R³_{b} und R³_{c} Cyclo-C₅-C₁₂-alkyl oder Phenyl und die anderen Reste Wasserstoff oder Methyl oder R³ₐ, R³_{b} und R³_{c} unabhängig voneinander Wasserstoff oder C₁-C₂₈-Alkyl bedeuten,
*in situ* in Gegenwart eines sauren Katalysators umsetzt und das Gemisch der Verbindungen (I) und (II) isoliert.

In einer besonders bevorzugten Ausführungsform der Erfindung werden die beiden Verfahrensschritte als Eintopfverfahren durchführt.

R³ und R^{3'} mit der Bedeutung C₂-C₃₀-Alkyl bedeutet vorzugsweise C₂-C₂₀-Alkyl, insbesondere C₄-C₁₂-Alkyl, z. B. geradkettiges oder verzweigtes (ab 3 C-Atomen) Alkyl, z. B. Ethyl, n-Propyl, Isopropyl, n-Butyl, Isobutyl, tert.-Butyl, n-Pentyl, Isopentyl, Neopentyl, n-Hexyl, 2-Ethyl-n-butyl, 1-Methyl-n-pentyl, 1,3-Dimethylbutyl, n-Heptyl, Isoheptyl, 1,1,3,3-Tetramethylbutyl, 1-Methylhexyl, 2-Methylhexyl, n-Octyl, Isooctyl, 1,4,4-Trimethyl-2-pentyl, 1-Methylheptyl, n-Nonyl, 1,1,3-Trimethylhexyl, n-Decyl, n-Undecyl, n-Dodecyl, 1-Methylundecyl, n-Dodecyl, n-Tetradecyl, n-Hexadecyl, n-Octadecyl oder n-Eicosyl.

Cyclo-C₅-C₁₂-alkyl-C₂-C₄-alkyl ist z. B. Cyclopentyl-1,1-ethyl, Cyclohexyl-1,1-ethyl, Cyclopentyl-1,2-ethyl, Cyclohexyl-1,2-ethyl, Cyclopentyl-1,2-propyl oder Cyclohexyl-1,2-propyl.

α-C₁-C₂-Alkylbenzyl ist z. B. Methylbenzyl (= 1,1-Phenethyl).

In einer bevorzugten Ausführungsform des Verfahrens wird die Umsetzung im ersten Verfahrensschritt mit ca. 0,5 bis 200 Mol% elementarem Schwefel in Gegenwart von ca. 0,001 bis 10 Mol% des Kondensationskatalysators aus der Gruppe Jod, Aluminiumbromid, Aluminiumchlorid, Eisen-III-Chlorid, Antimonchlorid, Kupferiodid und Schwefeliodid bei Temperaturen von ca. 80° bis 250°C durchgeführt.

In einer weiteren bevorzugten Ausführungsform des Verfahrens wird die Umsetzung mit 10 bis 150, insbesondere mit 15 bis 100 Mol%, elementarem Schwefel ausgeführt.

Zu den bevorzugten Ausführungsformen des Verfahrens gehört die Umsetzung mit 0,001 bis 1,0 Mol% des Kondensationskatalysators.

In einer besonders bevorzugten Ausführungsform des Verfahrens wird die Umsetzung mit 0,001 bis 0,01 Mol% elementarem lod durchgeführt.

Die Reaktionstemperatur liegt zwischen ca. 80° bis 250°C, bevorzugt zwischen 120° bis 190°C. Während der Reaktion bildet sich Schwefelwasserstoff (H₂S). Dieser wird wegen seiner Toxizität und Geruchsbelästigung aus dem Reaktionsgefäss entfernt und in eine wässrige Alkalihydroxidlösung geleitet. Dabei bildet sich das Alkalisulfid, das sich leicht entsorgen lässt.

Die Reaktionszeit kann im ersten Verfahrensschritt beispielsweise ca. 1 bis 15 Stunden betragen, wobei sich eine Reaktionszeit von 2 bis 4 Stunden als zweckmässig und eine solche von ca. 1 bis 2 Stunden als bevorzugt erweist.

Die Umsetzung lässt sich so ausführen, dass man das Diphenylamin (III), gegebenenfalls gelöst in einem der genannten Lösungsmittel, den Schwefel und den Katalysator vorlegt. Unter Rühren erhitzt man das Gemisch auf die angegebene Temperatur. Der Ablauf der Reaktion ist an der Bildung von Schwefelwasserstoff erkennbar. Nach der angegebenen Reaktionszeit oder wenn sich analytisch kein freier Schwefel mehr nachweisen lässt, kann man die Reaktion der ersten Verfahrensstufe beenden, z. B. indem man das Reaktionsgefäss auf ca. 100°C abkühlen lässt.

Das Gemisch der Verbindungen (III) und (IV) lässt sich im zweiten Verfahrensschritt in einem molaren Verhältnis von ca. 95 : 5 bis 5 : 95, insbesondere 30 : 70 bis 70 : 30, einsetzen.

In einer bevorzugten Variante des Verfahrens setzt man im zweiten Verfahrensschritt die Verbindungen (III) und Verbindungen (IV) in einem molaren Verhältnis von ca. 30 : 70 bis 5 : 95, besonders bevorzugt 20 : 80 bis 10 : 90, ein.

Das Olefin (V) wird in einem molaren Verhältnis von ca. 0,5 bis 4,0 Mol, insbesondere 1,0 bis 3,0 Mol, vor allem 1,0 bis 2,0 Mol, pro Mol der Mischungen aus den Verbindungen (III) und (IV) im zweiten Verfahrensschritt eingesetzt.

Die Anzahl der C-Atome im Olefin (V) entspricht der Anzahl der C-Atome in R³ oder R^{3'}. Wenn R³ oder R^{3'} tert.-Octyl oder 1,4,4-Trimethyl-2-pentyl bedeuten, verwendet man Diisobutylen als Olefin (V). Geeignete weitere Olefine sind z. B. Ethylen, Propylen, Isobutylen, 2-Methylpenten, Tripropylen, Tetrapropylen, Styrol oder Methylstyrol.

Als saure Katalysatoren eignen sich Protonendonatoren (sogenannte Brønsted-Säuren), Elektronenakzeptorverbindungen (sogenannte Lewis-Säuren), Kationenaustauscherharze, Alumosilicate oder natürlich vorkommende oder modifizierte Schichtsilicate.

Geeignete Protonendonatoren (sogenannte Brønsted-Säuren) sind z. B. salzbildende anorganische oder organische Säuren, z. B. Mineralsäuren wie Salzsäure, Schwefelsäure oder Phosphorsäure, Carbonsäuren, z. B. Essigsäure, oder Sulfonsäuren, z. B. Methansulfonsäure, Benzolsulfonsäure oder p-Toluolsulfonsäure. Besonders geeignet ist p-Toluolsulfonsäure.

Geeignete Elektronenakzeptorverbindungen (sogenannte Lewis-Säuren) sind z. B. Zinntetrachlorid, Zinkchlorid, Aluminiumchlorid oder Bortrifluoridetherat. Besonders geeignet sind Zinntetrachlorid und Aluminiumchlorid.

Geeignete Kationenaustauscherharze sind z. B. Styrol-Divinylbenzol-Copolymere mit Sulfosäuregruppen als lonenaustauscherfunktion, z. B. die bekannten Produkte Amberlite® und Amberlyst® von Rohm und Haas, z. B. AMBERLITE 200, oder Dowex® 50 von Dow Chemicals, perfluorierte lonenaustauscherharze, z. B. Nafion® H von DuPont, oder andere super-saure lonenaustauscherharze, z. B. die von *T. Yamaguchi in Applied Catalysis 61, 1-25 (1990),* oder *M.Hino et al.* in J. *Chem. Soc. Chemical Comm. 1980, 851-852* beschriebenen.

Geeignete Alumonsilicate sind z. B. amorphe Aluminiumsilicate, welche ca. 10-30% Siliciumdioxid und ca. 70-90% Aluminiumoxid enthalten und in der Petrochemie verwendet werden, z. B. Aluminiumsilicat HA-HPV® von Ketjen (Akzo), oder kristalline Aluminiumsilicate, z. B. sogenannte Zeolithe, welche als anorganische Kationenaustauscher, als sogenannte Molekularsiebe oder in der Petrochemie als sogenannte Cracking-Katalysatoren verwendet werden, z. B. Faujasite der Fa. Union Carbide, z. B. Zeolith R®, Zeolith Y® und ultrastabiler Zeolith; Zeolith Beta® und Zeolith ZSM-12® von der Fa. Mobil Oil; oder Zeolith Mordenit® der Fa. Norton.

Geeignete natürlich vorkommende Schichtsilicate werden auch als "Saure Erden" bezeichnet und sind z. B. mit Mineralsäuren, wie Schwefelsäure und/oder Salzsäure aktivierte, z. B. Montmorillonite, welche vorzugsweise einen Feuchtigkeitsgehalt unter 10 %, insbesondere unter 5 %, aufweisen, z. B. sogenannte Erden vom Typ Fuller, z. B. kommerziell unter den Bezeichnungen Fulcat® erhältliche Typen, z. B. die Typen 20, 22 A, 22 B und 40 (mit Schwefelsäure aktivierte Tonerden), Fulmont® (Laporte Industries), z. B. die Typen XMP-4, XMP-3, 700 C und 237, oder saure Erden der Typen K0 und K10 (mit Salzsäure aktiviert), KS und KSF (mit Schwefelsäure aktiviert) oder KSF0 (mit Salzsäure und Schwefelsäure aktiviert) der Fa. Südchemie, ferner Erden auf Bentonit-Basis, z. B. Produkte der Typen Filtrol® oder Retrol® (Engelhard Corp.).

In einer besonders bevorzugten Ausführungsform des Verfahrens verwendet man Fulcat® 22 B, ein säureaktiviertes Montmorrillonit mit 4% freier Feuchtigkeit und einem Säuretiter von 20 mg KOH/g.

Modifizierte Schichtsilicate werden auch als "Pillared Clays" bezeichnet und leiten sich von den weiter vorn beschriebenen natürlich vorkommenden Schichtsilicaten ab, indem sie zwischen den Silicatschichten noch Oxide von beispielsweise Zirkon, Eisen, Zink, Nickel, Chrom, Cobalt oder Magnesium oder Elementen der seltenen Erden enthalten. Modifizierte Schichtsilicate sind z. B. von J. *Clark et al.* in *J. Chem. Soc. Chem. Comm. 1989, 1353-1354* beschrieben. Besonders bevorzugte modifizierte Schichtsilicate sind z. B. die von der Fa. Contract Chemicals hergestellten Produkte Envirocat® EPZ-10, EPZG oder EPIC.

Der saure Katalysator kann beispielsweise in einer Menge von 1-50, insbesondere 5-25, ganz besonders 5-20 Gewichtsprozent oder wenn es sich um sogenannte Brønsted-Säuren oder Lewis-Säuren handelt, in einer Menge von 0,002 bis 10 Mol%, vorzugsweise 0,1 bis 5,0 Mol%, hinzugegeben werden.

Die Reaktionstemperatur im zweiten Reaktionsschritt liegt zwischen ca. 60° bis 250°C, bevorzugt zwischen 110° bis 200°C, besonders bevorzugt zwischen 160° bis 195°C.

Die Reaktion in beiden Reaktionsschritten kann mit oder bevorzugt ohne Lösungs- oder Verdünnungsmittel durchgeführt werden. Wenn man ein Lösungsmittel verwendet, sollte es unter den gegebenen Reaktionsbedingungen inert sein und einen geeignet hohen Siedepunkt haben. Geeignete Lösungsmittel sind beispielsweise gegebenenfalls halogenierte Kohlenwasserstoffe, polare aprotische Lösungsmittel, flüssige Amide und Alkohole. Beispielhaft seien genannt: Petroletherfraktionen, vorzugsweise höhersiedende, Toluol, Mesitylen, Dichlorbenzol, Tetrahydrofuran (THF), Dimethylformamid (DMF), Dimethylacetamid, (DMA), Hexamethylphosphorsäuretriamid (HMPTA), Glyme und Diglyme, Dimethylsulfoxid (DMSO), Tetramethylharnstoff (TMU), Alkohole, wie Butanol oder Ethylenglycol.

Der Ablauf der Verfahrensschritte lässt sich beispielhaft wie folgt illustrieren:
Herstellung von Gemischen aus alkyliertem Diphenylamin und Phenothiazin durch Umsetzung mit Diisobutylen:

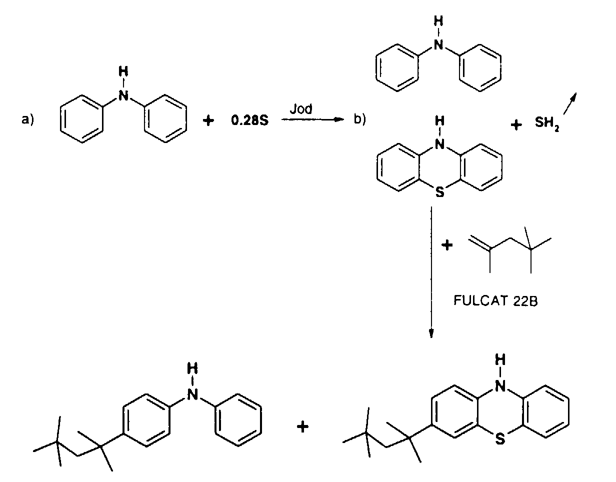

Die Erfindung betrifft bevorzugt ein Verfahren zur Herstellung eines Gemisches von alkylierten Phenothiazinen (I) und Diphenylaminen (II), welches dadurch gekennzeichnet ist, dass man ein Diphenylamin (III) mit elementarem Schwefel in Gegenwart eines Kondensationskatalysators aus der Gruppe Jod, Aluminiumbromid, Aluminiumchlorid, Eisen-III-Chlorid, Antimonchlorid, Kupferiodid und Schwefeliodid umsetzt und das erhältliche Stoffgemisch von Diphenylamin (III) und Phenothiazin (IV) mit einem die Anzahl der C-Atome in R³ oder R^{3'} enthaltenden Olefin (V), worin R³ₐ, R³_{b} und R³_{c} die genannten Bedeutungen haben, *in situ* in Gegenwart eines sauren Katalysators umsetzt und das Gemisch der Verbindungen (I) und (II) isoliert.

Die Erfindung betrifft besonders bevorzugt ein Verfahren zur Herstellung eines Gemisches von alkylierten Phenothiazinen (I) und Diphenylaminen (II), welches dadurch gekennzeichnet ist, dass man ein Diphenylamin (III) mit elementarem Schwefel in Gegenwart von lod als Kondensationskatalysator umsetzt und das erhältliche Stoffgemisch von Diphenylamin (III) und Phenothiazin (IV) mit einem die Anzahl der C-Atome in R³ oder R^{3'} enthaltenden Olefin (V), worin R³ₐ, R³_{b} und R³_{c} die genannten Bedeutungen haben, *in situ* in Gegenwart eines sauren Katalysators umsetzt und das Gemisch der Verbindungen (I) und (II) isoliert.

Eine weitere bevorzugte Ausführungsform betrifft ein Verfahren zur Herstellung eines Gemisches von alkylierten Phenothiazinen (I) und Diphenylaminen (II), worin R¹ und R² Wasserstoff, einer der Reste R³ und R^{3'} Wasserstoff und der andere 1,4,4-Trimethyl-2-pentyl oder Nonyl oder beide Reste R³ und R^{3'} 1,4,4-Trimethyl-2-pentyl oder Nonyl bedeuten, welches dadurch gekennzeichnet ist, dass man ein Diphenylamin (III), worin R¹ und R² Wasserstoff bedeuten, mit elementarem Schwefel in Gegenwart von lod als Kondensationskatalysator umsetzt und das erhältliche Stoffgemisch von Diphenylamin (III) und Phenothiazin (IV) mit Diisobutylen oder Tripropylen *in situ* in Gegenwart eines sauren Katalysators umsetzt und das Gemisch der Verbindungen (I) und (II) isoliert.

Die verfahrensgemäss erhältlichen Gemische von alkylierten Phenothiazinen (I) und Diphenylaminen (II) eignen sich zum Stabilisieren von organischen Verbindungen gegen lichtinduzierten, thermischen und/oder oxidativen Abbau. Die Gemische sind insbesondere in Kombination mit geeigneten Substraten als Stabilisatoren für synthetische, halbsynthetische oder natürliche Polymere, insbesondere thermoplastische Kunststoffe und Elastomere, sowie für funktionelle Flüssigkeiten, insbesondere Schmierstoffe, Metallbearbeitungs- und Hydraulikflüssigkeiten, verwendbar.

Die genannten funktionellen Flüssigkeiten, z. B. Schmierstoffzusammensetzungen, z. B. Fette, Getriebe-, Metallbearbeitungs- und Hydraulikflüssigkeiten, können zusätzlich weitere Additive enthalten, die zugegeben werden, um ihre Grundeigenschaften noch weiter zu verbessern. Dazu gehören: Antioxidantien, Metalldesaktivatoren, Rostinhibitoren, Viskositätsindex-Verbesserer, Stockpunkterniedriger, Dispergiermittel, Detergentien, Hochdruckzusätze und Antiverschleissadditive. Solche Additive gibt man in den jeweils dafür üblichen Mengen im Bereich von je etwa 0,01 bis 10,0 Gew.% zu.

Die genannten Komponenten können den Schmierstoffen auf an sich bekannte Weise beigemischt werden. Es ist auch möglich, ein Konzentrat oder ein sogenanntes Additivpaket herzustellen, das nach Massgabe des Verbrauchs auf Einsatzkonzentrationen für den entsprechenden Schmierstoff verdünnt werden kann.

Auf diese Weise verbesserte mineralische und synthetische Schmieröle, Schmierfette, hydraulische Flüssigkeiten und Elastomere zeigen ausgezeichnete Antioxidationseigenschaften, die durch stark reduzierte Alterungserscheinungen der zu schützende Teile deutlich werden. Besonders vorteilhaft sind die Mischungen in Schmierölen, worin sie eine ausgezeichnete Antioxidations- und Antikorrosionswirkung ohne Säure- oder Schlammbildung zeigen.

Die Einarbeitung in die für die jeweilige Anwendung vorgesehenen Materialien kann beispielsweise durch Einmischen oder Aufbringen des verfahrensgemäss erhältlichen Gemisches und gegebenenfalls weiterer Additive nach den üblichen Methoden erfolgen.

Die folgenden Beispiele illustrieren die Erfindung:

### Beispiel 1

1.1. 507,7 g (3,0 mol) Diphenylamin und 26,75 g (0,834 Mol) Schwefel werden in einem auf 195°C vorgeheizten Ölbad vorgelegt. Bei ca. 100°C Innentemperatur werden 0,1 g (0,0016 mol) Jod zugegeben und die Temperatur weiter erhöht. Nach 15 Minuten Reaktionszeit ist die Temperatur von 175°C erreicht, wobei Schwefelwasserstoff sich abzuspalten beginnt, den man durch Einleiten in eine 5%-ige wässrige Natriumhydroxidlösung vernichtet. Die Temperatur wird 45 min. bei 185°C thermostatisiert. Nach Kontrolle im Dünnschichtchromatogramm (DC) ist im braunen Reaktionsgemisch kein Schwefel mehr nachweisbar.
1.2. Den Ansatz aus Verfahrensschritt 1.1. lässt man auf ca. 100°C abkühlen. Man gibt 132 g (1,176 mol) Diisobutylen und 50 g FULCAT 22B hinzu und heizt den Ansatz am Wasserabscheider bis zum starken Rückfluss wieder auf, wobei ca. 9 ml Wasser abgespalten werden. Nach 30 Minuten Reaktionszeit steigert sich die Innentemperatur auf 184°C. Man lässt 396,5 g (3,534 Mol) weiteres Diisobutylen so zutropfen dass die Temperatur nicht unter 165°C sinkt (Zutropfzeit 5 ½ Stunden). Anschliessend lässt man das Gemisch bei konstanten Temperaturbedingungen (170-175°C) 2 Stunden lang ausreagieren.
1.3. Man lässt den Ansatz auf ca. 50°C abkühlen und verdünnt diesen mit 400 ml Hexan. Man filtriert den Katalysator ab und engt das gelbliche Filtrat im Rotationsverdampfer ein. Das unreagierte Diphenylamin wird aus dem gelblichen Öl im Vakuum bei 10⁻² mbar bei 150°C Badtemperatur innerhalb 3 Stunden abdestilliert. Man erhält 867,3 g eines leicht gelbbraunen klaren Öls.

### Charakterisierung

Elementaranalyse: 4,25 % N; 1,29 % S. Zusammensetzung des Gemisches im Gaschromatogramm siehe Tabelle.

### Beispiel 2

Durch Zugabe einer grösseren Menge an Schwefel können Mischungen mit einem höheren Phenothiazingehalt hergestellt werden. Man geht analog Beispiel 1 vor und setzt 48,1 g (1,5 Mol) Schwefel ein. Man erhält 893 g eines leicht gelbbraunen klaren Öls.

### Charakterisierung

Elementaranalyse: 4,38 % N; 2,75 S%; Zusammensetzung des Gemisches im Gaschromatogramm siehe Tabelle.

### Beispiel 3

Durch Zugabe einer grösseren Menge an Schwefel und Alkylierung mit einer grösseren Menge Diisobutylen können Mischungen mit einem höheren Phenothiazingehalt und höherem Alkylierungsgrad hergestellt werden. Man geht analog Beispiel 1 vor und setzt 144,3 g (4,5 Mol) Schwefel und 158 g Diisobutylen ein. Man erhält 928 g eines leicht gelbbraunen klaren Öls.

### Charakterisierung

Elementaranalyse: 4,19 % N; 7,07 % S; Zusammensetzung des Gemisches im Gaschromatogramm siehe Tabelle.

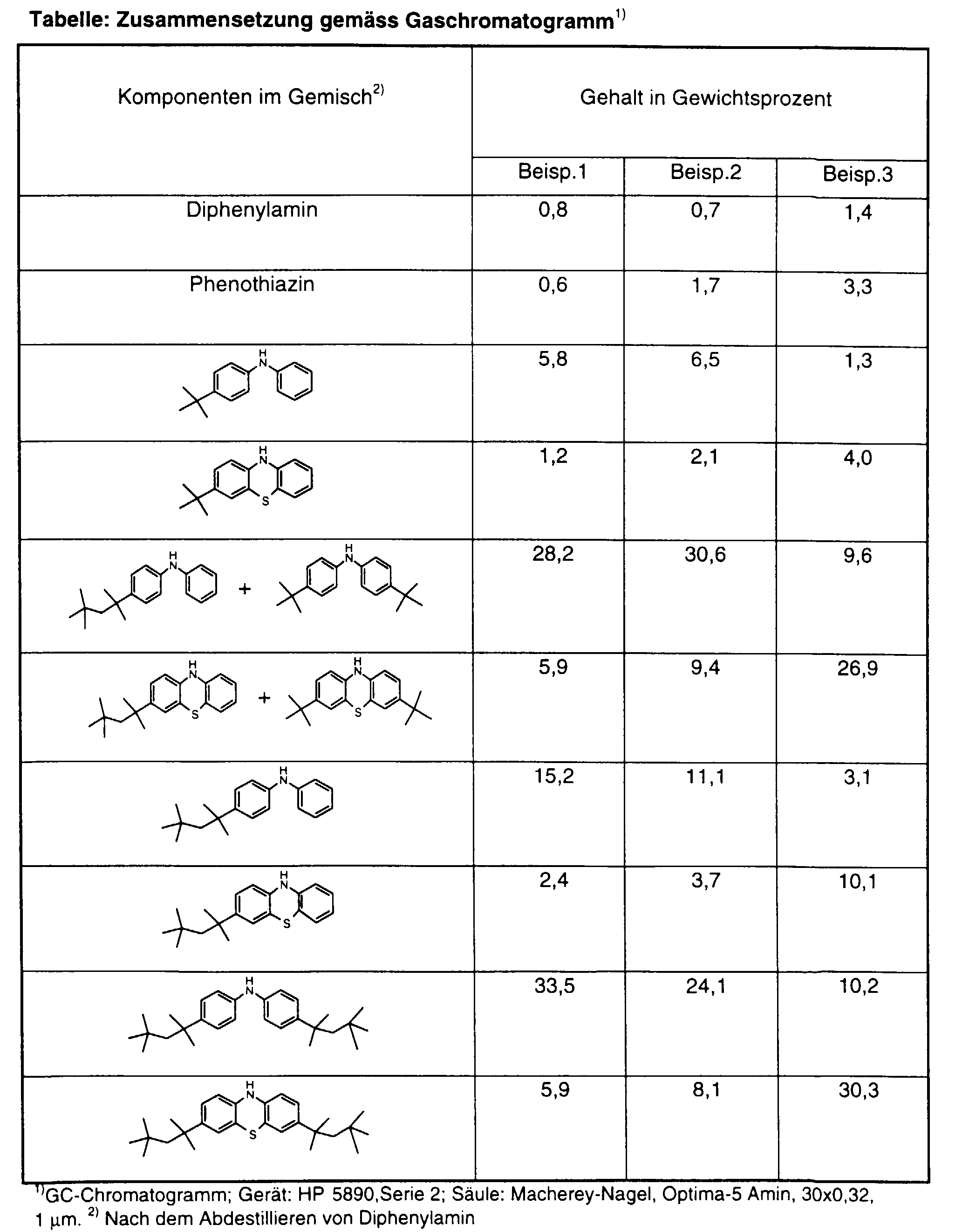

### Beispiel 4

4.1. 338,45 g (2,0 Mol) Diphenylamin und 96,2 g (3 Mol) Schwefel werden in einem auf 195°C vorgeheizten Ölbad vorgelegt. Bei ca. 100°C Innentemperatur werden 0,1 g (0,0016 mol) Jod zugegeben und die Temperatur weiter erhöht. Nach 40 Minuten Reaktionszeit sind 175°C erreicht, wobei Schwefelwasserstoff sich abzuspalten beginnt, den man durch Einleiten in eine 5%-ige wässrige Natriumhydroxidlösung vernichtet. Die Temperatur wird 45 min. bei 185°C thermostatisiert.
4.2. Den Ansatz aus Verfahrensschritt 1.1. lässt man auf ca. 100°C abkühlen. Man gibt 250 g Tripropylen (Nonen) und 35 g FULCAT 22B hinzu und heizt den Ansatz am Wasserabscheider bis zum starken Rückfluss wieder auf, wobei ca. 9 ml Wasser abgespalten werden. Nach 30 Minuten Reaktionszeit steigert sich die Innentemperatur auf 164°C. Anschliessend werden 760 g Tripropylen innerhalb 3 Stunden zugetropft. Die Temperatur fällt bis auf 143°C. Anschliessend lässt man bei konstanten Temperaturbedingungen (143-145°C) 6 Stunden lang ausreagieren.
4.3. Man lässt den Ansatz auf ca. 50°C abkühlen und verdünnt diesen mit 400 ml Hexan. Man filtriert den Katalysator ab und engt das gelbliche Filtrat im Rotationsverdampfer ein. Das unreagierte Tripropylen wird aus dem gelblichen Filtrat im Rotationsverdampfer entfernt. Das braune Öl wird im Vakuum bei 10⁻² mbar und 80°C Badtemperatur innerhalb 3 Stunden getrocknet. Man erhält 721,6 g eines braunen klaren Öls.

### Charakterisierung

Elementaranalyse: 4,28 % N; 6,56 %S.

## Patentansprüche

1. Verfahren zur Herstellung eines Gemisches enthaltend alkylierte Phenothiazine der Formel: 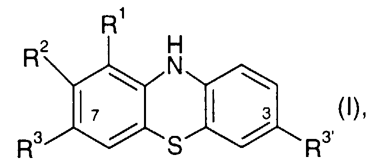 und entsprechend substituierte Diphenylamine der Formel: 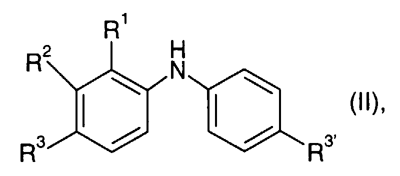 worin R¹ und R² Wasserstoff oder zusammen die Gruppe: 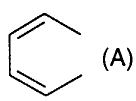 darstellen;
einer der Reste R³ und R^{3'} Wasserstoff und der andere C₂-C₃₀-Alkyl, Cyclo-C₅-C₁₂-alkyl-C₂-C₄-alkyl, α-C₁-C₂-Alkylbenzyl oder α,α-Dimethylbenzyl bedeutet; oder beide Reste R³ und R^{3'} C₂-C₃₀-Alkyl, Cyclo-C₅-C₁₂-alkyl-C₂-C₄-alkyl, α-C₁-C₂-Alkylbenzyl oder
α,α-Dimethylbenzyl bedeuten, wenn R¹ und R² Wasserstoff darstellen; oder
R³ Wasserstoff und R^{3'} C₂-C₃₀-Alkyl, Cyclo-C₅-C₁₂-alkyl-C₂-C₄-alkyl, α-C₁-C₂-Alkylbenzyl oder α,α- Dimethylbenzyl bedeuten, wenn R¹ und R² zusammen die Gruppe A darstellen,
dadurch gekennzeichnet, dass man ein Diphenylamin der Formel: 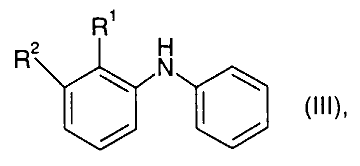 worin R¹ und R² die unter der Formel I genannten Bedeutungen haben, mit elementarem Schwefel in Gegenwart eines Kondensationskatalysators aus der Gruppe Jod, Aluminiumbromid, Aluminiumchlorid, Eisen-III-Chlorid, Antimonchlorid, Kupferiodid und Schwefeliodid umsetzt und das erhältliche Stoffgemisch von Diphenylamin (III) und Phenothiazin der Formel: 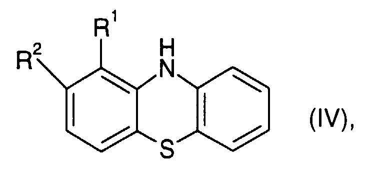 worin R¹ und R² die unter der Formel I genannten Bedeutungen haben, mit einem die Anzahl der C-Atome in R³ oder R^{3'} enthaltenden Olefin der Formel: 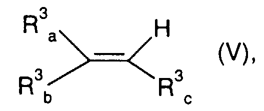 worin einer der Reste R³ₐ, R³_{b} und R³_{c} Cyclo-C₅-C₁₂-alkyl oder Phenyl und die anderen Reste Wasserstoff oder Methyl oder R³ₐ, R³_{b} und R³_{c} unabhängig voneinander Wasserstoff oder C₁-C₂₈-Alkyl bedeuten,
*in situ* in Gegenwart eines sauren Katalysators umsetzt und das Gemisch der Verbindungen (I) und (II) isoliert.

2. Verfahren gemäss Anspruch 1 zur Herstellung eines Gemisches von alkylierten Phenothiazinen (I) und Diphenylaminen (II), dadurch gekennzeichnet, dass man ein Diphenylamin (III) mit elementarem Schwefel in Gegenwart eines Kondensationskatalysators aus der Gruppe Jod, Aluminiumbromid, Aluminiumchlorid, Eisen-III-Chlorid, Antimonchlorid, Kupferiodid und Schwefeliodid umsetzt und das erhältliche Stoffgemisch von Diphenylamin (III) und Phenothiazin (IV) mit einem die Anzahl der C-Atome in R³ oder R^{3'} enthaltenden Olefin (V), worin R³ₐ, R³_{b} und R³_{c} die genannten Bedeutungen haben, *in situ* in Gegenwart eines sauren Katalysators umsetzt und das Gemisch der Verbindungen (I) und (II) isoliert.

3. Verfahren gemäss Anspruch 1 zur Herstellung eines Gemisches von alkylierten Phenothiazinen (I) und Diphenylaminen (II), dadurch gekennzeichnet, dass man ein Diphenylamin (III) mit elementarem Schwefel in Gegenwart von lod als Kondensationskatalysator umsetzt und das erhältliche Stoffgemisch von Diphenylamin (III) und Phenothiazin (IV) mit einem die Anzahl der C-Atome in R³ oder R^{3'} enthaltenden Olefin (V), worin R³ₐ, R³_{b} und R³_{c} die genannten Bedeutungen haben, *in situ* in Gegenwart eines sauren Katalysators umsetzt und das Gemisch der Verbindungen (I) und (II) isoliert.

4. Verfahren gemäss Anspruch 1 zur Herstellung eines Gemisches von alkylierten Phenothiazinen (I) und Diphenylaminen (II), worin R¹ und R² Wasserstoff, einer der Reste R³ und R^{3'} Wasserstoff und der andere 1,4,4-Trimethyl-2-pentyl oder beide Reste R³ und R^{3'} 1,4,4-Trimethyl-2-pentyl bedeuten, dadurch gekennzeichnet, dass man ein Diphenylamin (III), worin R¹ und R² Wasserstoff bedeuten, mit elementarem Schwefel in Gegenwart von lod als Kondensationskatalysator umsetzt und das erhältliche Stoffgemisch von Diphenylamin (III) und Phenothiazin (IV) mit Diisobutylen *in situ* in Gegenwart eines sauren Katalysators umsetzt und das Gemisch der Verbindungen (I) und (II) isoliert.

5. Verfahren gemäss Anspruch 1 zur Herstellung eines Gemisches von alkylierten Phenothiazinen (I) und Diphenylaminen (II), worin R¹ und R² Wasserstoff, einer der Reste R³ und R^{3'} Wasserstoff und der andere 1,4,4-Trimethyl-2-pentyl oder Nonyl oder beide Reste R³ und R^{3'} 1,4,4-Trimethyl-2-pentyl oder Nonyl bedeuten, dadurch gekennzeichnet, dass man ein Diphenylamin (III), worin R¹ und R² Wasserstoff bedeuten, mit elementarem Schwefel in Gegenwart von lod als Kondensationskatalysator umsetzt und das erhältliche Stoffgemisch von Diphenylamin (III) und Phenothiazin (IV) mit Diisobutylen oder Tripropylen *in situ* in Gegenwart von aktiven Katalysatoren auf Schichtsilicat-Basis, Montmorrilloniten oder Erden auf der Basis von Bentoniten umsetzt und das Gemisch der Verbindungen (I) und (II) isoliert.

6. Verfahren gemäss Anspruch 1 zur Herstellung eines Gemisches von alkylierten Phenothiazinen (I) und Diphenylaminen (II), dadurch gekennzeichnet, dass man die beiden Verfahrensschritte als Eintopfverfahren durchführt.
